# EUROPEAN PATENT APPLICATION

(11) **EP 4 741 437 A1**
(43) Date of publication of application: **13.05.2026**
(21) Application number: 24836092.7
(22) Date of filing: 04.07.2024
(51) Int. Cl.: C08G 18/65, A41D 3/04, A41D 13/00, A41D 31/00, A41D 31/102, A61F 13/51, C08G 18/00, C08G 18/40, C08G 18/44, D06N 3/14, D06N 7/00, E04H 15/00

(54) **POLYURETHANE RESIN COMPOSITION**

(30) Priority: 05.07.2023 JP 2023110618
(71) Applicant: Mitsubishi Chemical Corporation, Tokyo 100-8251 (JP)
(72) Inventor: SHIRAI, Kazuaki, Tokyo 100-8251 (JP); OHARA, Teruhiko, Tokyo 100-8251 (JP); MORI, Atsushi, Tokyo 100-8251 (JP)
(74) Representative: Ter Meer Steinmeister & Partner
(86) International application number: PCT/JP2024/024165
(87) International publication number: WO 2025/009580

(57) **Abstract**

Provided is a polyurethane resin composition containing a polyurethane resin (U) and an organic solvent (S), in which the polyurethane resin (U) contains a structural unit derived from a polyoxyethylene diol (A), a structural unit derived from a polycarbonate diol (B), a structural unit derived from an aliphatic isocyanate compound (C), a structural unit derived from a diol (D) having 2 to 6 carbon atoms, and a structural unit derived from a diamine (E) having 2 to 20 carbon atoms, and the structural unit derived from the aliphatic isocyanate compound (C) is a structural unit derived from an acyclic aliphatic diisocyanate (c1) in which two isocyanate groups are directly bonded to an acyclic hydrocarbon having an odd number of carbon atoms, or a structural unit derived from an alicyclic diisocyanate (c2) in which the number of isocyanate groups directly bonded to an alicyclic hydrocarbon is 0 or 1.

## Description

### TECHNICAL FIELD

The present disclosure relates to: a polyurethane resin composition; and a moisture-permeable waterproof fabric, a sportswear, a rainwear, a tent, and a hygiene product, in which the polyurethane resin composition is used.

### BACKGROUND ART

Polyurethane resins have excellent elasticity and durability (wear resistance and heat resistance) and are thus used in a variety of materials and, in recent years, because of their excellent properties, they are expected to be developed as moisture-permeable waterproof materials in particular.

The moisture-permeable waterproof materials are used in outdoor products, sportswear, sports shoes, rainwear (e.g., raincoats), tents, disposable diapers, wound dressings (bandages), and the like, and the polyurethane resins used for these applications are strongly desired to have a good balance of physical properties, such as durability, in addition to moisture-permeable waterproof performance.

As a polyurethane resin for a moisture-permeable waterproof material, Patent Document 1 proposes a polyurethane resin formed from a polyurethane resin composition for a moisture-permeable waterproof fabric, which composition is composed of an alcohol solution of a hydrophilic polyurethane resin having an oxyethylene group content of 10 to 80% by weight in the resin, and a polyisocyanate.

Further, Patent Document 2 proposes an aqueous polyurethane resin that has a structural unit derived from an emulsifier having a polyoxyethylene group as a side chain.

Moreover, Patent Document 3 proposes a polyurethane resin obtained by curing a composition that contains a monofunctional epoxy compound and a polyurethane obtained by reacting one or more polyols selected from polytetramethylene polyols, polycarbonate polyols, and polyolefin polyols, with a polyisocyanate, a carboxy group-containing polyol, and a chain extender.

### PRIOR ART DOCUMENTS

### PATENT DOCUMENTS

Patent Document 1: JP 2007-211239 A
Patent Document 2: JP 2006-335951 A
Patent Document 3: WO 2021/187504 A1

### SUMMARY OF INVENTION

### TECHNICAL PROBLEMS

An object of the present disclosure is to provide a polyurethane resin composition that can give a moisture-permeable waterproof material excellent in moisture-permeable waterproof performance and durability.

Another object of the present disclosure is to provide a moisture-permeable waterproof fabric, a sportswear, a rainwear, a tent, and a hygiene product, in which the polyurethane resin composition is used.

### SOLUTION TO PROBLEMS

The present inventors intensively studied to solve the above-described problems and consequently discovered that a moisture-permeable waterproof material excellent in moisture-permeable waterproof performance and durability can be obtained from a polyurethane resin composition that contains a polyurethane resin having specific structure and composition and an organic solvent; and that the polyurethane resin composition is suitable for use in moisture-permeable waterproof fabrics, sportswear, rainwear, tents, hygiene products, and the like. That is, the present disclosure has the following characteristics.

[1] A polyurethane resin composition, comprising:
   a polyurethane resin (U); and
   an organic solvent (S),
   wherein
   the polyurethane resin (U) comprises a structural unit derived from a polyoxyethylene diol (A), a structural unit derived from a polycarbonate diol (B), a structural unit derived from an aliphatic isocyanate compound (C), a structural unit derived from a diol (D) having 2 to 6 carbon atoms, and a structural unit derived from a diamine (E) having 2 to 20 carbon atoms, and
   the structural unit derived from the aliphatic isocyanate compound (C) comprises a structural unit derived from an acyclic aliphatic diisocyanate (c1) in which two isocyanate groups are directly bonded to an acyclic hydrocarbon having an odd number of carbon atoms, or a structural unit derived from an alicyclic diisocyanate (c2) in which the number of isocyanate groups directly bonded to an alicyclic hydrocarbon is 0 or 1.
[2] The polyurethane resin composition according to [1], wherein, in the polyurethane resin (U), a weight ratio (A)/((A) + (B)) of the structural unit derived from the polyoxyethylene diol (A) relative to a total weight of the structural unit derived from the polyoxyethylene diol (A) and the structural unit derived from the polycarbonate diol (B) is from 0.50 to 0.90.
[3] The polyurethane resin composition according to [1] or [2], wherein a molar ratio of isocyanate group-derived nitrogen atoms in the structural unit derived from the aliphatic isocyanate compound (C) relative to the number of moles of amino group-derived nitrogen atoms in the diamine (E) is from 1.5 to 2.5.
[4] The polyurethane resin composition according to any one of [1] to [3], wherein the structural unit derived from the polyoxyethylene diol (A) is a structural unit derived from a polyoxyethylene diol having a hydroxyl value of from 26.0 to 140.0 mgKOH/g.
[5] The polyurethane resin composition according to any one of [1] to [4], wherein the structural unit derived from the polycarbonate diol (B) comprises a structural unit derived from a polycarbonate diol having a hydroxyl value of from 32.0 to 124.7 mgKOH/g.
[6] The polyurethane resin composition according to any one of [1] to [5], wherein
   the structural unit derived from the polycarbonate diol (B) comprises a structural unit represented by the following Formula (b1) and a structural unit represented by the following Formula (b2), or the structural unit represented by the following Formula (b2), and when both the structural units of Formula (b1) and Formula (b2) are present,
   a molar ratio (b1)/(b2) is from 95/5 to 5/95:

      -O-R¹-O- (b1)

      -O-R²-O- (b2)
   in Formula (b1), R¹ represents a divalent alkylene group having 3 to 5 carbon atoms which may have a substituent; and, in Formula (b2), R² represents a divalent alkylene group having 6 to 20 carbon atoms which may have a substituent.
[7] The polyurethane resin composition according to any one of [1] to [6], wherein a content of the structural unit derived from the aliphatic isocyanate compound (C) in the polyurethane resin (U) is 50% by weight or less.
[8] The polyurethane resin composition according to any one of [1] to [7], wherein the diamine (E) comprises a diamine having 6 to 12 carbon atoms.
[9] The polyurethane resin composition according to any one of [1] to [8], wherein, in the polyurethane resin (U), a molar ratio of hydroxy group-derived oxygen atoms of the polyoxyethylene diol (A) and hydroxy group-derived oxygen atoms of the polycarbonate diol (B) relative to a number of moles of hydroxy group-derived oxygen atoms of the diol (D) is from 0.5 to 1.5.
[10] The polyurethane resin composition according to any one of [1] to [9], wherein, in the polyurethane resin (U), a molar ratio of isocyanate group-derived nitrogen atoms of the aliphatic isocyanate compound (C) relative to a total number of moles of hydroxy group-derived oxygen atoms of the polyoxyethylene diol (A), hydroxy group-derived oxygen atoms of the polycarbonate diol (B), and hydroxy group-derived oxygen atoms of the diol (D) is from 1.5 to 4.0.
[11] The polyurethane resin composition according to any one of [1] to [10], wherein a content of a polar solvent (Y) satisfying the following conditions (y1) and (y2) in the organic solvent (S) is 5% by weight or less:
   (y1) Not classified into the carcinogenicity category according to the GHS (9th revised edition) classification; and
   (y2) The acute toxicity is classified into Categories 1 to 3 according to the GHS (9th revised edition) classification, or the reproductive toxicity is classified into Category 1A or 1B according to the GHS (9th revised edition) classification.
[12] The polyurethane resin composition according to any one of [1] to [11], wherein the organic solvent (S) comprises a monohydric alcohol having 5 or fewer carbon atoms, and at least one selected from the group consisting of a ketone having 6 or fewer carbon atoms and an ester having 8 or fewer carbon atoms.
[13] The polyurethane resin composition according to any one of [1] to [12], wherein a content of the polyurethane resin (U) is from 5 to 50% by weight.
[14] The polyurethane resin composition according to any one of [1] to [13], further comprising a biomass-derived compound.
[15] A moisture-permeable waterproof fabric comprising the polyurethane resin composition according to any one of [1] to [14].
[16] A sportswear comprising the polyurethane resin composition according to any one of [1] to [14].
[17] A rainwear comprising the polyurethane resin composition according to any one of [1] to [14].
[18] A tent comprising the polyurethane resin composition according to any one of [1] to [14].
[19] A hygiene product comprising the polyurethane resin composition according to any one of [1] to [14].

### ADVANTAGEOUS EFFECTS OF INVENTION

According to the present disclosure, a polyurethane resin composition that can give a moisture-permeable waterproof material excellent in moisture-permeable waterproof performance and durability can be provided.

Further, by using the polyurethane resin composition, it is possible to provide a moisture-permeable waterproof fabric, a sportswear, a rainwear, a tent, and a hygiene product.

### DESCRIPTION OF EMBODIMENTS

Embodiments of the present disclosure will now be described in detail; however, the following descriptions of requirements are merely examples (representative examples) of the embodiments of the present disclosure, and the present disclosure is not limited thereto within the scope of the gist of the present disclosure.

In the present specification, those ranges expressed with "to" are intended to each include the numerical values or physical values stated before and after "to". Further, those numerical values or physical values that are stated as upper and lower limits are intended to be included in the respective ranges.

One embodiment of the present disclosure is a polyurethane resin composition that contains a polyurethane resin (U) and an organic solvent (S). The polyurethane resin (U) has a structural unit derived from a polyoxyethylene diol (A), a structural unit derived from a polycarbonate diol (B), a structural unit derived from an aliphatic isocyanate compound (C), a structural unit derived from a diol (D) having 2 to 6 carbon atoms, and a structural unit derived from a diamine (E) having 2 to 20 carbon atoms, and the structural unit derived from the aliphatic isocyanate compound (C) is a structural unit derived from an acyclic aliphatic diisocyanate (c1) in which two isocyanate groups are directly bonded to an acyclic hydrocarbon having an odd number of carbon atoms, or a structural unit derived from an alicyclic diisocyanate (c2) in which the number of isocyanate groups directly bonded to an alicyclic hydrocarbon is 0 or 1.

### [1. Polyurethane Resin (U)]

The polyurethane resin (U) has a structural unit derived from a polyoxyethylene diol (A), a structural unit derived from a polycarbonate diol (B), a structural unit derived from an aliphatic isocyanate compound (C), a structural unit derived from a diol (D) having 2 to 6 carbon atoms, and a structural unit derived from a diamine (E) having 2 to 20 carbon atoms.

The polyurethane resin (U) may be used singly, or in any combination of two or more kinds thereof at any ratio.

The content of the polyurethane resin (U) in the polyurethane resin composition is preferably from 5 to 50% by weight, more preferably from 10 to 50% by weight. When the content of the polyurethane resin (U) is in this range, the viscosity can be easily adjusted at the time of application so that the ease of handling is improved.

### [1-1. Structural Unit Derived From Polyoxyethylene Diol (A)]

The polyoxyethylene diol (A) that gives the structural unit derived from the polyoxyethylene diol (A) may be a diol whose repeating units consist of oxyethylene units, and may have other units within a range that does not hinder the effects of the present disclosure. Examples of other units include units derived from the below-described isocyanate compound (c).

The polyoxyethylene diol (A) may be used singly, or in any combination of two or more kinds thereof at any ratio.

Further, a polyoxyethylene diol obtained via biomass ethylene by ring-opening polymerization of biomass ethylene oxide using biomass ethanol as a crude raw material is preferred because it is of biomass origin.

The polyoxyethylene diol (A) has a number average molecular weight Mn of preferably from 800 to 4,300, more preferably from 1,000 to 3,000, and still more preferably from 1,600 to 2,400.

The polyoxyethylene diol (A) has a hydroxyl value of preferably from 26.0 to 140.0 mgKOH/g, more preferably from 26.0 to 125.0 mgKOH/g, still more preferably from 28.0 to 75.0 mgKOH/g, and particularly preferably from 40.0 to 70.0 mgKOH/g.

The hydroxyl value of the polyoxyethylene diol (A) is measured in accordance with Method A (a method using an acetylation reagent) of JIS K1557-1:2007.

When the hydroxyl value of the polyoxyethylene diol (A) is equal to or more than the above-described lower limit, a moisture-permeable waterproof material having good texture and moisture permeability can be provided. Further, when the hydroxyl value of the polyoxyethylene diol (A) is equal to or less than the above-described upper limit, a polyurethane resin (U) having excellent durability can be obtained.

The polyoxyethylene diol (A) has a CPR value (controlled polymerization rate), which is defined in JIS K1557-4 (2007), of preferably 5 or less, more preferably 3 or less, and still more preferably 1 or less. When the CPR value is 5 or less, the polyurethane reaction can be easily controlled particularly in the two-step method of the present disclosure, and gelation can be inhibited.

The polyoxyethylene diol (A) may be a polyoxyethylene diol (Ac) that is a copolymer of a low-molecular-weight polyoxyethylene diol (a) and an isocyanate compound (c).

The low-molecular-weight polyoxyethylene diol (a) is a diol whose repeating units consist of oxyethylene units. The term "low-molecular-weight" used herein means that the number average molecular weight (Mn) is 1,500 or less.

The low-molecular-weight polyoxyethylene diol (a) may be used singly, or in any combination of two or more kinds thereof at any ratio.

The low-molecular-weight polyoxyethylene diol (a) has a hydroxyl value of preferably from 75.0 to 450.0 mgKOH/g, more preferably from 80.0 to 375.0 mgKOH/g, still more preferably from 90.0 to 225.0 mgKOH/g, yet still more preferably from 100.0 to 225.0 mgKOH/g, and particularly preferably from 100.0 to 150.0 mgKOH/g. The hydroxyl value of the low-molecular-weight polyoxyethylene diol (a) is measured in accordance with Method A (a method using an acetylation reagent) of JIS K1557-1:2007.

When the hydroxyl value of the low-molecular-weight polyoxyethylene diol (a) is equal to or more than the above-described lower limit, a moisture-permeable waterproof material having good texture and moisture permeability can be provided. Further, when the hydroxyl value of the polyoxyethylene diol (A) is equal to or less than the above-described upper limit, a polyurethane resin (U) having excellent durability can be obtained.

Examples of the isocyanate compound (c) include the below-described aliphatic isocyanate compound (C) and aromatic isocyanate compound (cr), and the aromatic isocyanate compound (cr) is preferred.

The isocyanate compound (c) may be used singly, or in any combination of two or more kinds thereof at any ratio.

Specific examples of the aromatic isocyanate compound (cr) include xylylene diisocyanate, 4,4'-diphenyl diisocyanate, 2,4-tolylene diisocyanate, 2,6-tolylene diisocyanate, m-phenylene diisocyanate, p-phenylene diisocyanate, 4,4'-diphenylmethane diisocyanate, 4,4'-diphenyldimethylmethane diisocyanate, 4,4'-dibenzyl diisocyanate, dialkyldiphenylmethane diisocyanate, tetraalkyldiphenylmethane diisocyanate, 1,5-naphthylene diisocyanate, 3,3'-dimethyl-4,4'-biphenylene diisocyanate, polymethylene polyphenylisocyanate, phenylene diisocyanate, and m-tetramethylxylylene diisocyanate.

Among them, from the standpoint of obtaining a good balance between the durability and the moisture permeability of the resulting polyurethane resin (U), the aromatic isocyanate compound (cr) is preferably selected from 2,4-tolylene diisocyanate, 2,6-tolylene diisocyanate, and 4,4'-diphenylmethane diisocyanate.

The use of the aromatic isocyanate compound (cr) in the reaction with the low-molecular-weight polyoxyethylene diol (a) can improve the cohesive force of the resulting polyurethane resin (U) and increase the strength; however, it may cause yellowing. Therefore, when producing a polyoxyethylene diol (Ac) by reacting the low-molecular-weight polyoxyethylene diol (a) and the aromatic isocyanate compound (cr), from the standpoint of attaining both satisfactory strength and inhibition of yellowing, the amount of the aromatic isocyanate compound (cr) to be used is preferably from 5 to 30% by weight particularly preferably from 10 to 25% by weight, relative to a total amount of the aliphatic isocyanate compound (C) and the aromatic isocyanate compound (cr) that are used in the reaction with the polyoxyethylene diol (A).

### [1-2. Structural Unit Derived From Polycarbonate Diol (B)]

The polycarbonate diol (B) that gives the structural unit derived from the polycarbonate diol (B) has hydroxy groups at both ends of the molecule and a carbonate bond in the main chain.

The polycarbonate diol (B) has a hydroxyl value of preferably from 32.0 to 124.7 mgKOH/g, more preferably from 35.0 to 119.3 mgKOH/g, still more preferably from 36.0 to 75.0 mgKOH/g, yet still more preferably from 40.0 to 100.0 mgKOH/g, and particularly preferably from 45.0 to 90.0 mgKOH/g.

When the hydroxyl value of the polycarbonate diol (B) is equal to or more than the above-described lower limit, a moisture-permeable waterproof material having good texture and moisture permeability can be provided. Further, when the hydroxyl value of the polycarbonate diol (B) is equal to or less than the above-described upper limit, a polyurethane resin (U) having excellent strength can be obtained.

The structural unit derived from the polycarbonate diol (B) preferably has either or both of a structural unit represented by the following Formula (b1) and a structural unit represented by the following Formula (b2), and more preferably has a structural unit represented by the following Formula (b1) and a structural unit represented by the following Formula (b2), or the structural unit represented by the following Formula (b2). Further, when both the structural units of Formula (b1) and Formula (b2) are present, a molar ratio (b1)/(b2) of these structural units is usually from 100/0 to 0/100, preferably from 95/5 to 5/95.

-O-R¹-O- (b1)

-O-R²-O- (b2)

(in Formula (b1), R¹ represents a divalent alkylene group having 3 to 5 carbon atoms which may have a substituent; and, in Formula (b2), R² represents a divalent alkylene group having 6 to 20 carbon atoms which may have a substituent).

Examples of a diol that gives the structural unit represented by Formula (b1) include 2-methyl-1,3-propanediol and 1,4-butanediol.

Among them, from the standpoint of obtaining a polyurethane resin (U) having excellent durability and strength, the diol that gives the structural unit represented by Formula (b1) is preferably 1,4-butanediol.

The diol that gives the structural unit represented by Formula (b1) may be used singly, or in any combination of two or more kinds thereof at any ratio.

In Formula (b2), R² is preferably a divalent alkylene group having 8 to 20 carbon atoms which may have a substituent, more preferably a divalent alkylene group having 8 to 12 carbon atoms which may have a substituent. It is also preferred that these alkylene groups be unsubstituted.

Examples of a diol that gives the structural unit represented by Formula (b2) include 1,6-hexanediol, 1,8-octanediol, 2-methyl-1,8-octanediol, 1,9-nonanediol, 1,10-decanediol, 1,11-undecanediol, 1,12-dodecanediol, 1,13-tridecanediol, 1,14-tetradecanediol, 1,16-hexadecanediol, 1,18-octadecanediol, 1,12-octadecanediol, and 1,20-eicosanediol.

Among them, from the standpoint of obtaining an excellent balance between the strength and durability of the polyurethane resin (U) and the texture of the moisture-permeable waterproof material, the diol that gives the structural unit represented by Formula (b2) is preferably 1,9-nonanediol, 1,10-decanediol, 1,11-undecanediol, or 1,12-dodecanediol, more preferably selected from 1,10-decanediol, 1,11-undecanediol, and 1,12-dodecanediol, and still more preferably 1,10-decanediol.

The diol that gives the structural unit represented by Formula (b2) may be used singly, or in any combination of two or more kinds thereof at any ratio.

From the standpoint of reducing the environmental load, the structural unit represented by Formula (b1) is preferably derived from a plant. One example of a diol that gives the structural unit represented by Formula (b1) and can be applied as a plant-derived diol is 1,4-butanediol.

From the standpoint of reducing the environmental load, the structural unit represented by Formula (b2) is preferably derived from a plant. Examples of a diol that gives the structural unit represented by Formula (b2) and can be applied as a plant-derived diol include 1,9-nonanediol, 1,10-decanediol, 1,11-undecanediol, 1,12-dodecanediol, 1,13-tridecanediol, 1,12-octadecanediol, and 1,20-eicosanediol.

The molar ratio (b1)/(b2) of the structural unit represented by Formula (b1) and the structural unit represented by Formula (b2) is usually from 100/0 to 0/100, preferably from 95/5 to 5/95, more preferably from 89/11 to 5/95, still more preferably from 85/15 to 5/95, and particularly preferably from 75/25 to 5/95.

When the molar ratio (b1)/(b2) is in the above-described range, the resulting polyurethane resin (U) has good strength and durability. Further, by controlling the molar ratio (b1)/(b2) to be in the above-described preferred range, a polyurethane resin composition having superior storage stability and coatability can be provided.

The polycarbonate diol (B) has poor compatibility with the polyoxyethylene diol (A); therefore, it is also necessary to use a combination having good compatibility.

### [1-3. Structural Unit Derived From Aliphatic Isocyanate Compound (C)]

The aliphatic isocyanate compound (C) that gives the structural unit derived from the aliphatic isocyanate compound (C) is an acyclic aliphatic diisocyanate (c1) in which two isocyanate groups are directly bonded to an acyclic hydrocarbon having an odd number of carbon atoms, or an alicyclic diisocyanate (c2) in which the number of isocyanate groups directly bonded to an alicyclic hydrocarbon is 0 or 1.

The aliphatic isocyanate compound (C) may be used singly, or in any combination of two or more kinds thereof at any ratio.

Examples of the acyclic aliphatic diisocyanate (c1) include 1,5-pentamethylene diisocyanate, 2,2,4-trimethylhexamethylene diisocyanate, 2,4,4-trimethylhexamethylene diisocyanate, and 1,7-heptamethylene diisocyanate.

Examples of the alicyclic diisocyanate (c2) include isophorone diisocyanate, 1,3-bis(isocyanatomethyl)cyclohexane, and norbornene diisocyanate.

Among them, from the standpoint of the balance between the solubility and the durability of the resulting polyurethane resin (U), the aliphatic isocyanate compound (C) is preferably selected from 1,5-pentamethylene diisocyanate, 1,7-heptamethylene diisocyanate, and isophorone diisocyanate. Further, from the standpoint of the solubility of the resulting polyurethane resin, the aliphatic isocyanate compound (C) is preferably the alicyclic diisocyanate (c2), and more preferably isophorone diisocyanate.

The content of the structural unit derived from the aliphatic isocyanate compound (C) in the polyurethane resin (U) is preferably from 10 to 50% by weight, more preferably from 20 to 40% by weight, and still more preferably from 25 to 33% by weight.

By controlling the content of the structural unit derived from the aliphatic isocyanate compound (C) to be in the above-described range, a good balance between the solubility and the strength of the polyurethane resin (U) is obtained.

### [1-4. Structural Unit Derived From Diol (D) Having 2 to 6 Carbon Atoms]

Examples of the diol (D) having 2 to 6 carbon atoms, which gives the structural unit derived from the diol (D) having 2 to 6 carbon atoms, include ethylene glycol, 1,3-propanediol, 1,4-butanediol, 1,5-pentanediol, 1,6-hexanediol, 2-methyl-1,3-propanediol, and 2,2-dimethyl-1,3-propanediol.

Among them, from the standpoint of obtaining good strength of the resulting polyurethane resin (U), ethylene glycol, 1,3-propanediol, and 1,4-butanediol are preferred.

The diol (D) may be used singly, or in any combination of two or more kinds thereof at any ratio.

### [1-5. Diamine (E) Having 2 to 20 Carbon Atoms]

The diamine (E) having 2 to 20 carbon atoms, which gives the structural unit derived from the diamine (E) having 2 to 20 carbon atoms, is preferably a diamine having 6 to 12 carbon atoms.

Examples of the diamine (E) include ethylenediamine, 1,3-diaminopropane, hexamethylenediamine, isophoronediamine, and 4,4'-diaminodicyclohexylmethane.

Among them, from the standpoint of obtaining good strength of the resulting polyurethane resin (U), the diamine (E) is preferably ethylenediamine or isophoronediamine. Further, from the standpoint of obtaining a good balance between the strength and the solubility in solvents of the resulting polyurethane resin (U), the diamine (E) is more preferably isophoronediamine.

The diamine (E) may be used singly, or in any combination of two or more kinds thereof at any ratio.

### [1-6. Composition of Polyurethane Resin (U)]

In the polyurethane resin (U), a weight ratio (A)/((A) + (B)) of the structural unit derived from the polyoxyethylene diol (A), relative to a total weight of the structural unit derived from the polyoxyethylene diol (A) and the structural unit derived from the polycarbonate diol (B), is preferably from 0.50 to 0.95, more preferably from 0.50 to 0.90, still more preferably higher than 0.50 but 0.90 or lower, yet still more preferably from 0.53 to 0.90, and particularly preferably from 0.55 to 0.85.

When the ratio (A)/((A) + (B)) is in the above-described range, excellent moisture-permeable waterproof performance can be exerted. Further, when the ratio (A)/((A) + (B)) is equal to or higher than the above-described lower limit, the polyurethane resin (U) can be ensured with moisture permeability, solubility in the organic solvent (S), and strength.

In the polyurethane resin (U), a ratio ([OH](A) + [OH] (B))/[OH] (D) of a total number of moles of hydroxy group-derived oxygen atoms of the polyoxyethylene diol (A) and hydroxy group-derived oxygen atoms of the polycarbonate diol (B), relative to the number of moles of hydroxy group-derived oxygen atoms of the diol (D), is preferably from 0.5 to 1.5, and more preferably from 1.0 to 1.5. When the ratio ([OH] (A) + [OH] (B))/[OH] (D) is in this range, the polyurethane resin (U) has a good balance of the solubility in solvents, the strength, and the moisture permeability.

In the polyurethane resin (U), a ratio [NCO]/[OH] of the number of moles of isocyanate group-derived nitrogen atoms of the aliphatic isocyanate compound (C), relative to a total number of moles of hydroxy group-derived oxygen atoms of the polyoxyethylene diol (A), hydroxy group-derived oxygen atoms of the polycarbonate diol (B), and hydroxy group-derived oxygen atoms of the diol (D) is preferably from 1.5 to 4.0, more preferably from 1.5 to 3.0, and still more preferably from 1.8 to 2.5. When the ratio [NCO]/[OH] is in this range, the polyurethane resin (U) has a good balance between the solubility in solvents and the strength.

In the polyurethane resin (U), a ratio [NCO]/[NH₂] of the number of moles of isocyanate group-derived nitrogen atoms of the aliphatic isocyanate compound (C), relative to the number of moles of amino group-derived nitrogen atoms of the diamine (E), is preferably from 1.5 to 4.0, more preferably from 1.5 to 2.5, and still more preferably from 1.8 to 2.5.

By controlling the ratio [NCO]/[NH₂] to be in the above-described range, the durability of the polyurethane resin (U) is improved. When the ratio [NCO]/[NH₂] is equal to or higher than the above-described lower limit, the polyurethane resin (U) can be provided with good flexibility, solubility, and texture. Further, when the ratio [NCO]/[NH₂] is equal to or lower than the above-described upper limit, not only the brittleness of the polyurethane resin (U) can be improved, but also the cohesive force and the strength of the polyurethane resin (U) can be improved.

### [1-7. Moisture-Permeable Waterproof Performance]

The polyurethane resin (U) has a moisture permeability of preferably from 50,000 to 200,000 g/m²/24h, more preferably from 60,000 to 200,000 g/m²/24h.

The moisture permeability of the polyurethane resin (U) is measured by molding the polyurethane resin (U) into a 20 µm-thick film and using this film as a test piece in accordance with Method B-1 (Potassium Acetate Method) of JIS L1099:2021 (revised on August 20, 2021).

When the moisture permeability of the polyurethane resin (U) is equal to or higher than the above-described lower limit, a moisture-permeable waterproof material having sufficient moisture permeability can be produced from a polyurethane resin composition containing the polyurethane resin (U).

Further, since the polyurethane resin (U) has good strength, a moisture-permeable waterproof material having excellent water pressure resistance, i.e., a moisture-permeable waterproof material having sufficient waterproof performance, can be produced from a polyurethane resin composition containing the polyurethane resin (U).

### [1-8. Weight-Average Molecular Weight]

The polyurethane resin (U) has a weight average molecular weight (Mw) of preferably from 10,000 to 300,000, more preferably from 30,000 to 200,000, and still more preferably from 50,000 to 150,000.

When the weight average molecular weight of the polyurethane resin (U) is equal to or more than the above-described lower limit, sufficient strength can be obtained. When the weight average molecular weight of the polyurethane resin (U) is equal to or less than the above-described upper limit, the polyurethane resin composition can be easily handled.

The weight average molecular weight of the polyurethane resin (U) is a value in terms of polystyrene that is measured by gel permeation chromatography (GPC), and a specific measurement method is as described below in the section of Examples.

The value of weight average molecular weight/number average molecular weight (Mw/Mn), which is the molecular weight distribution of the polyurethane resin (U), is preferably from 1.5 to 3.5, more preferably from 1.7 to 3.0, and still more preferably from 2.0 to 2.5. When the molecular weight distribution is equal to or less than the above-described upper limit, high-molecular-weight substances in the polyurethane resin can be reduced so that the solubility of the polyurethane resin (U) in a solvent can be improved, while, when the molecular weight distribution is equal to or more than the above-described lower limit, oligomers can be reduced so that good strength and tensile strength can be obtained.

### [1-9. Method of Producing Polyurethane Resin (U)]

As a method of producing the polyurethane resin (U), any known polyurethane resin production method can be applied.

Specific examples thereof include: a one-step method in which the polyoxyethylene diol (A), the polycarbonate diol (B), the aliphatic isocyanate compound (C), the diol (D) having 2 to 6 carbon atoms, and the diamine (E) having 2 to 20 carbon atoms are mixed all at once and allowed to react; and a two-step method in which the polyoxyethylene (A), the polycarbonate diol (B), and the aliphatic isocyanate compound (C) are allowed to react first so as to prepare a prepolymer having isocyanate groups at both ends, and this prepolymer is subsequently allowed to react with the diol (D) having 2 to 6 carbon atoms and the diamine (E) having 2 to 20 carbon atoms.

The method of producing the polyurethane resin (U) is preferably the two-step method since it allows urethane bonds and urea bonds to be dispersed in the resulting polymer in a well-balanced manner. The polyurethane resin (U) obtained by the two-step method has a uniform dispersion of force against tension and exhibits good solubility in the organic solvent (S).

When the polyurethane resin (U) is produced by a solution method, it is preferred to use a reaction solvent having a low toxicity to the human body. Examples of the reaction solvent having a low toxicity to the human body include methyl ethyl ketone (MEK), ethyl acetate, and butyl acetate.

When the polyurethane resin (U) is produced by a solution method and a reaction solvent is used as the organic solvent (S) as is, the reaction solvent may contain the below-described polar solvent (Y); however, preferably, the reaction solvent does not contain the polar solvent (Y). More specifically, the content of the polar solvent (Y) in the reaction solvent is preferably from 0 to 40% by weight, more preferably from 0 to 30% by weight, still more preferably from 0 to 20% by weight, yet still more preferably from 0 to 10% by weight, particularly preferably from 0 to 5% by weight, and especially preferably 0% by weight (i.e., the reaction solvent does not contain the polar solvent (Y)).

In the production of the polyurethane resin (U), when the sum of a total number of hydroxy groups in the polyoxyethylene diol (A) and the polycarbonate diol (B), the number of hydroxy groups in the diol (D) having 2 to 6 carbon atoms, and the number of amino groups in the diamine (E) having 2 to 20 carbon atoms is assumed to be 1.0 equivalent, the amount of the aliphatic isocyanate compound (C) to be used is preferably from 0.7 to 3.0 equivalents, more preferably from 0.8 to 2.0 equivalents, and still more preferably from 0.9 to 1.5 equivalents.

When the amount of the aliphatic isocyanate compound (C) to be used is equal to or more than the above-described lower limit, the molecular weight of the resulting polyurethane resin (U) is sufficiently large, so that the polyurethane resin (U) has good strength. When the amount of the aliphatic isocyanate compound (C) to be used is equal to or less than the above-described upper limit, a polyurethane resin (U) having a high solubility is obtained so that a moisture-permeable waterproof material having good texture can be produced from a polyurethane resin composition containing the polyurethane resin (U).

Further, in the production of the polyurethane resin (U), the molar amount of isocyanate groups in the aliphatic isocyanate compound (C), relative to the molar amount of amino groups in the diamine (E), is preferably from 1.5 to 4.0, more preferably from 1.5 to 2.5, and still more preferably from 1.8 to 2.5.

When the molar amount of isocyanate groups in the aliphatic isocyanate compound (C) is in the above-described range, a polyurethane resin (U) having excellent durability can be obtained. When the molar amount of isocyanate groups in the aliphatic isocyanate compound (C) is equal to or more than the above-described lower limit, since the amount of urea bonds is not excessively large, a polyurethane resin (U) having good flexibility, solubility, and texture can be obtained. Further, when the molar amount of isocyanate groups in the aliphatic isocyanate compound (C) is equal to or less than the above-described upper limit, since the amount of urethane bonds is not excessively large, a polyurethane resin (U) having good cohesive force and strength with low brittleness can be obtained.

In the production of the polyurethane resin (U), known chain terminators, catalysts, additives, and the like may be used as required.

### [Chain Terminator]

In the production of the polyurethane resin, a chain terminator that has one active hydrogen group, or one amino group and an active hydrogen group other than the amino group, may be used as required for the purpose of controlling the molecular weight of the polyurethane resin (U).

Examples of the chain terminator include: aliphatic monools, such as methanol, ethanol, propanol, butanol, and hexanol; aliphatic monoamines, such as diethylamine, dibutylamine, and n-butylamine; and alkanolamines, such as monoethanolamine, diethanolamine, 2-propanolamine, and morpholine.

These chain terminators may be used singly, or in any combination of two or more kinds thereof at any ratio.

### [Catalyst]

In a polyurethane-forming reaction performed in the production of the polyurethane resin (U), a known urethane polymerization catalyst typified by an amine-based catalyst, an organic metal salt, or the like may be used as well.

Examples of the amine-based catalyst include triethylamine, N-ethylmorpholine, and triethylenediamine.

Examples of the organic metal salt include: tin-based compounds, such as trimethyl tin laurate, dibutyl tin dilaurate, dioctyl tin dilaurate, and dioctyl tin dineodecanoate; and titanium-based compounds, such as tetrabutoxytitanate.

These catalysts may be used singly, or in any combination of two or more kinds thereof at any ratio.

### [2. Organic Solvent (S)]

The organic solvent (S) contained in the polyurethane resin composition according to the present embodiment is not particularly limited as long as it is an organic solvent capable of dissolving the polyurethane resin; however, the organic solvent (S) preferably contains at least one solvent selected from the group consisting of a ketone-based solvent, an ester-based solvent, and an alcohol-based solvent, and more preferably is at least one solvent selected from the group consisting of a ketone-based solvent, an ester-based solvent, and an alcohol-based solvent. It is also preferred that the organic solvent (S) be a solvent having a low toxicity to the human body and/or a solvent having a low environmental load. In recent years, to realize a sustainable society, there is an increasing demand for environmentally friendly products that do not expose workers, product users, and the like to a solvent that is highly toxic to the human body, and this is because there is a strong global demand not only for products that have a low content of a solvent that is highly toxic to the human body, but also for products that do not use a solvent that is highly toxic to the human body in the production process.

In the present specification, the term "dissolve" means that, when a solution is prepared by mixing a material with a solvent to a solid concentration of 30% by weight at a temperature of 25°C, the appearance of the solution is transparent after a lapse of 24 hours from the preparation.

In the present specification, the "solvent having a low toxicity to the human body" is a solvent not corresponding to a solvent that is highly toxic to the human body, meaning a solvent that does not fall under the acute toxicity categories 1 to 3, or a solvent that is not recognized as carcinogenic. The acute toxicity categories and solvents recognized as carcinogenic are defined in the GHS (Globally Harmonized System of Classification and Labelling of Chemicals) classification that was recommended by the United Nations in July 2003 and has been regularly updated since then and, in the present specification, GHS refers to the 9th edition. The GHS classification is available to the public on the website of the Ministry of Health, Labor and Welfare of Japan (https://anzeninfo.mhlw.go.jp/user/anzen/kag/ghs_symbol.html).

In the present specification, the "solvent having a low environmental load" is a solvent that requires a small amount of thermal energy for its volatilization and is highly effective in reducing carbon dioxide emissions, which solvent has a low boiling point of lower than 100°C, not a boiling point of 100°C or higher as in the case of water and amide-based organic solvents. It is noted here that, since these solvents have low solubility for polyurethane resins, their use in polyurethane resin compositions has conventionally often been avoided.

Examples of the ketone-based solvent include acetone, MEK, 2-pentanone, 3-pentanone, methyl isobutyl ketone, diisobutyl ketone, methyl isopropyl ketone, diacetone alcohol, cyclohexanone, and isophorone.

Among them, from the standpoint of the solubility and the evaporation rate of the polyurethane resin (U), the ketone-based solvent is preferably a ketone having 6 or fewer carbon atoms, more preferably MEK.

The ketone-based solvent may be used singly, or in any combination of two or more kinds thereof at any ratio.

Examples of the ester-based solvent include methyl acetate, ethyl acetate, n-propyl acetate, isopropyl acetate, n-butyl acetate, sec-butyl acetate, methoxybutyl acetate, amyl acetate, methyl lactate, ethyl lactate, and butyl lactate.

Among them, from the standpoint of the solubility and the evaporation rate of the polyurethane resin (U), the ester-based solvent is preferably an ester having 8 or fewer carbon atoms, more preferably selected from ethyl acetate and n-butyl acetate.

The ester-based solvent may be used singly, or in any combination of two or more kinds thereof at any ratio.

Examples of the alcohol-based solvent include methanol, ethanol, n-butanol, isobutanol, tert-butanol, sec-butanol, isopropyl alcohol, n-propyl alcohol, and 2-ethylhexanol.

Among them, from the standpoint of the solubility of the polyurethane resin (U), the alcohol-based solvent is preferably a monohydric alcohol having 5 or fewer carbon atoms, more preferably isopropyl alcohol.

The alcohol-based solvent may be used singly, or in any combination of two or more kinds thereof at any ratio.

From the standpoint of the solubility of the polyurethane resin (U), the organic solvent (S) is preferably a mixed solvent of a ketone-based solvent and an alcohol-based solvent, and the ratio of the ketone-based solvent and the alcohol-based solvent (ketone-based solvent/alcohol-based solvent) is preferably from 70/30 to 95/5 in terms of weight ratio. Further, from the standpoint of the balance between the solubility of the polyurethane resin (U) and the drying properties upon application, the organic solvent (S) preferably contains a monohydric alcohol having 5 or fewer carbon atoms, and at least one selected from the group consisting of a ketone having 6 or fewer carbon atoms and an ester having 8 or fewer carbon atoms.

The organic solvent (S) may contain a polar solvent (Y) that satisfies the following (y1) and (y2); however, since the polar solvent (Y) is highly toxic to the human body, the content of the polar solvent (Y) in the organic solvent (S) is preferably low, and it is more preferred that the organic solvent (S) does not contain the polar solvent (Y). Many of the solvents known to have high solubility for general polyurethane resins correspond to the polar solvent (Y); however, since the polyurethane resin (U) according to the present embodiment can be dissolved in the above-described solvent having a low toxicity, it is not necessary to select a highly toxic solvent such as the polar solvent (Y) as the organic solvent (S). Therefore, it is possible to set the content of the polar solvent (Y) in the organic solvent (S) to a low level. Examples of a solvent having a high solubility for polyurethane resins include solvents having a polar term (δP) of 11.0 or more in Hansen solubility parameters.
(y1) Not classified into the carcinogenicity category according to the GHS (9th revised edition) classification; and
(y2) The acute toxicity is classified into Categories 1 to 3 according to the GHS (9th revised edition) classification, or the reproductive toxicity is classified into Category 1A or 1B according to the GHS (9th revised edition) classification.

The polar solvent (Y) may further satisfy the following (y3):
(y3) The polar term (δP) in Hansen solubility parameters is 11.0 or more.

More specifically, from the standpoint of the solubility of the polyurethane resin (U) as well as the standpoint of the burden on the human body and the environment, the content of the polar solvent (Y) in the organic solvent (S) is preferably from 0 to 40% by weight, more preferably from 0 to 30% by weight, still more preferably from 0 to 20% by weight, yet still more preferably from 0 to 10% by weight, particularly preferably from 0 to 5% by weight, especially preferably from 0 to 1% by weight, and most preferably 0% by weight (i.e., the polyurethane resin composition does not contain the polar solvent (Y)).

Examples of the polar solvent (Y) include: compounds having an amide group, such as dimethylformamide (DMF), dimethylacetamide, N-methylpyrrolidone, and N-ethylpyrrolidone; and compounds having a nitrile group, such as acetonitrile.

### [3. Additives]

The polyurethane resin composition according to the present embodiment may contain various additives, such as a biomass-derived compound, a heat stabilizer, an ultraviolet absorber, a light stabilizer, a colorant, a flame retardant, and an inorganic filler, within a range that does not impair the properties of the polyurethane resin (U).

The additives may be used singly, or in any combination of two or more kinds thereof at any ratio.

The biomass-derived compound is used for the purpose of improving the bio-based degree of a product.

Examples of the biomass-derived compound include plant-derived fiber powders, such as grape-derived fiber powder, pineapple-derived fiber powder, apple fruit-derived fiber powder, cactus-derived fiber powder, and pulp-derived fiber powder.

The biomass-derived compound may be used singly, or in any combination of two or more kinds thereof at any ratio.

Examples of the heat stabilizer include: phosphorus compounds, such as aliphatic, aromatic, or alkyl group-substituted aromatic esters of phosphoric acid or phosphorous acid, hypophosphorous acid derivatives, phenylphosphonic acid, phenylphosphinic acid, diphenylphosphonic acid, polyphosphonates, dialkyl pentaerythritol diphosphites, and dialkyl bisphenol A diphosphites; phenol derivatives, particularly hindered phenol compounds; sulfur-containing compounds, such as thioethers, dithioacid salts, mercaptobenzimidazoles, thiocarbanilides, and thiodipropionic acid esters; and tin compounds, such as tin maleate and dibutyl tin monoxide.

The heat stabilizer may be used singly, or in any combination of two or more kinds thereof at any ratio.

The ultraviolet absorber is not particularly limited, and any known additive can be selected. Examples thereof include "TINUVIN 328" and "TINUVIN 234" (which are manufactured by Ciba Specialty Chemicals Holding Inc.).

The light stabilizer is not particularly limited, and any known additive can be selected. Examples thereof include ADK STAB LA-77 and ADK STAB LA-57 (which are manufactured by ADEKA Corporation).

The heat stabilizer, the ultraviolet absorber, and the light stabilizer may be used singly, or in any combination of two or more kinds thereof at any ratio, and are each preferably of a high-molecular-weight type that hardly bleeds out from the polyurethane resin.

Examples of the colorant include: dyes, such as direct dyes, acid dyes, basic dyes, and metal complex salt dyes; inorganic pigments, such as carbon black, titanium oxide, zinc oxide, iron oxide, and mica; and organic pigments, such as coupling azo-based, condensed azo-based, anthraquinone-based, thioindigo-based, dioxazone-based, and phthalocyanine-based pigments.

The colorant may be used singly, or in any combination of two or more kinds thereof at any ratio.

Examples of the flame retardant include additive-type or reactive-type flame retardants, such as organic compounds containing phosphorus and halogen, organic compounds containing bromine or chlorine, ammonium polyphosphate, aluminum hydroxide, and antimony oxide.

The flame retardant may be used singly, or in any combination of two or more kinds thereof at any ratio.

Examples of the inorganic filler include short glass fibers, carbon fibers, alumina, talc, graphite, melamine, and white clay.

The inorganic filler may be used singly, or in any combination of two or more kinds thereof at any ratio.

The additives may be added to the raw materials or reaction system at any stage in the production of the polyurethane resin (U), or may be added to the polyurethane resin (U) and the organic solvent (S) at the time of mixing these materials to prepare the polyurethane resin composition.

The content of the additives in the polyurethane resin composition is preferably from 0.01 to 10% by weight, more preferably from 0.05 to 5% by weight, and still more preferably from 0.1 to 1% by weight, relative to a total amount of the polyurethane resin (U).

When the content of the additives is equal to or higher than the above-described lower limit, the effects of the additives can be obtained sufficiently. Further, when the content of the additives is equal to or lower than the above-described upper limit, precipitation of the additives is unlikely to occur.

### [4. Use]

The polyurethane resin composition according to the present embodiment can be utilized in various products that require moisture permeability and waterproof performance.

For example, a moisture-permeable waterproof composite material that has a polyurethane resin layer containing the polyurethane resin (U) and a substrate layer adjacent to the polyurethane resin layer can be produced by applying the polyurethane resin composition to at least one surface of a substrate and drying the solvent. It is noted here that a method of producing the moisture-permeable waterproof composite material is not limited to the above-described method.

Further, when the substrate is a fibrous substrate, the moisture-permeable waterproof composite material is a moisture-permeable waterproof fabric and can be applied to fabric products having moisture permeability and waterproof performance.

The base material of the fibrous substrate may be any of cellulosic fibers such as cotton, linen, and rayon, and synthetic fibers such as polyester, polyamide, and polyolefin.

As the form of the fibrous substrate, any of a woven fabric, a knitted fabric, a nonwoven fabric, and the like can be used; however, the fibrous substrate is preferably a woven fabric or a knitted fabric.

The moisture-permeable waterproof fabric can be suitably used for, for example: clothes, such as outdoor wear for mountain climbing, fishing, cycling, and the like, ski-related wear, windbreakers, sportswear, rainwear, athletic wear, golf-related wear, and outdoor workwear; tents; and mountain climbing-related materials, such as tarps.

Further, the polyurethane resin composition according to the present embodiment can be suitably used in hygiene products, for example, disposable diapers and wound dressings such as adhesive bandages and bandages.

### EXAMPLES

The present disclosure will now be described in more detail by way of Examples; however, the present invention is not limited by the below-described Examples within the gist of the present disclosure.

In the following Examples and Comparative Examples, various physical properties were measured by the below-described respective methods.

### [Evaluation Methods]

### (1) Molecular Weight

A polyurethane resin was dissolved in dimethylacetamide at a concentration of 0.14% by weight to obtain a dimethylacetamide solution. The thus-obtained dimethylacetamide solution was injected into a GPC apparatus [manufactured by Tosoh Corporation, product name "HLC-8220GPC" (columns: TSKgel GMHXL × 2)], and the weight average molecular weight (Mw) of the polyurethane resin was measured in terms of standard polystyrene.

### (2) Strength (Tensile Test)

A polyurethane resin solution was applied onto a fluororesin sheet (manufactured by Nitto Denko Corporation, product name "NITOFLON No. 900", thickness: 0.1 mm) using a 10-mil applicator, and the applied solution was dried at 60°C for 20 minutes and then at 80°C for 30 minutes to obtain a coating film. Subsequently, the thus-obtained coating film was left to stand under a constant temperature and humidity of 23°C and 55%RH for at least 12 hours to obtain a polyurethane film. From this polyurethane film, a 7 cm × 1 cm piece was cut out as a test piece.

For the thus-obtained test piece, using a benchtop-type precision universal tester (manufactured by Shimadzu Corporation, product name "AUTOGRAPH AGX-1kNX"), a tensile test was conducted in accordance with JIS K6251:2010, except that the shape of the test piece was changed to a rectangular shape, at a chuck distance of 30 mm, a tensile speed of 200 mm/min, a temperature of 23°C, and a relative humidity of 55%, and the tensile stress (100% modulus) at 100% elongation of the test piece was measured.

The strength was evaluated as follows based on the 100% modulus.
B: Less than 3.0 MPa
A: 3.0 MPa or more, but less than 5.0 MPa
S: 5.0 MPa or more

### (3) Tensile Strength (Breaking Strength)

A polyurethane resin solution was applied onto a fluororesin sheet (manufactured by Nitto Denko Corporation, product name "NITOFLON No. 900", thickness: 0.1 mm) using a 10-mil applicator, and the applied solution was dried at 60°C for 20 minutes and then at 80°C for 30 minutes to obtain a coating film. Subsequently, the thus-obtained coating film was left to stand under a constant temperature and humidity of 23°C and 55%RH for at least 12 hours to obtain a polyurethane film. From this polyurethane film, a 7 cm × 1 cm test piece was cut out.

For the thus-obtained test piece, using a benchtop-type precision universal tester (manufactured by Shimadzu Corporation, product name "AUTOGRAPH AGX-1kNX"), a tensile test was conducted in accordance with JIS K6251:2010, except that the shape of the test piece was changed to a rectangular shape, at a chuck distance of 30 mm, a tensile speed of 200 mm/min, a temperature of 23°C, and a relative humidity of 55%, and the tensile stress (tensile strength) at break of the test piece was measured.

The tensile strength was evaluated as follows.
B: Less than 17.0 MPa
A: 17.0 MPa or more, but less than 25.0 MPa
S: 25.0 MPa or more

### (3) Moisture Permeability

A polyurethane resin solution was applied onto a fluororesin sheet (manufactured by Nitto Denko Corporation, product name "NITOFLON No. 900", thickness: 0.1 mm) using a 4.5-mil applicator, and the applied solution was dried at 60°C for 20 minutes and then at 80°C for 30 minutes to obtain a 20 µm-thick polyurethane resin film.

Using this film, a moisture permeability test was conducted in accordance with Method B-1 (Potassium Acetate Method) of JIS L1099:2021, and the moisture permeability per 24 hours was calculated from the thus-obtained moisture permeation amount.

The moisture permeability was evaluated as follows.
B: Less than 70,000 g/m²/24h
A: 70,000 g/m²/24h or more, but less than 120,000 g/m²/24h
S: 120,000 g/m²/24h to 200,000 g/m²/24h

### (4) Storage Stability

A polyurethane resin solution was filled into a glass bottle that was subsequently sealed. After storing this glass bottle at room temperature (23°C) for one week, the appearance of the polyurethane resin solution was observed.

The storage stability was evaluated as follows.
B: The polyurethane resin solution was observed with a marked change such as generation of foreign matters or discoloration.
A: The polyurethane resin solution was observed with a slight change such as cloudiness, foreign matters, or discoloration.
S: The polyurethane resin solution had no change in appearance.

### (5) Coatability

A polyurethane resin solution was applied onto a fluororesin sheet (manufactured by Nitto Denko Corporation, product name "NITOFLON No. 900", thickness: 0.1 mm) using a 4.5-mil applicator, and the resulting coating film was observed immediately after the application.

The coatability was evaluated as follows.
B: Cissing occurred at 10 or more spots on the coating film.
A: Cissing occurred at less than 10 spots on the coating film.
S: No cissing was observed on the coating film.

### [Raw Materials, etc.]

PEG2000: polyoxyethylene diol having a number average molecular weight of 2,000 (hydroxyl value: 56.1 mgKOH/g) [manufactured by Sanyo Chemical Industries, Ltd.]
PEG1000: polyoxyethylene diol having a molecular weight of 1,000 (hydroxyl value: 112.1 mgKOH/g) [manufactured by Sanyo Chemical Industries, Ltd.]
NL2030DB: 1,4-butanediol/1,10-decanediol copolymerized polycarbonate diol having a number average molecular weight of 2,000 (hydroxyl value: 56.1 mgKOH/g) (1,4-butanediol/1,10-decanediol = 70/30) [manufactured by Mitsubishi Chemical Corporation]
NL2000B: 1,4-butanediol polymerized polycarbonate diol having a number average molecular weight of 2,000 (hydroxyl value: 57.6 mgKOH/g) [manufactured by Mitsubishi Chemical Corporation]
UH200: 1,6-hexanediol polymerized polycarbonate diol having a number average molecular weight of 2,000 (hydroxyl value: 57.5 mg KOH/g) [manufactured by UBE Corporation]
NL2010DB: 1,4-butanediol/1,10-decanediol copolymerized polycarbonate diol having a number average molecular weight of 2,000 (hydroxyl value: 56.3 mgKOH/g) (1,4-butanediol/1,10-decanediol = 90/10) [manufactured by Mitsubishi Chemical Corporation]
NL2070DB: 1,4-butanediol/1,10-decanediol copolymerized polycarbonate diol having a number average molecular weight of 2,000 (hydroxyl value: 56.7 mgKOH/g) (1,4-butanediol/1,10-decanediol = 29/71) [manufactured by Mitsubishi Chemical Corporation]
IPDI: isophorone diisocyanate [manufactured by FUJIFILM Wako Pure Chemical Corporation]
HDI: 1,6-hexamethylene diisocyanate [manufactured by Tokyo Chemical Industry Co., Ltd.]
H12MDI: 4,4'-dicyclohexylmethane diisocyanate [manufactured by Tokyo Chemical Industry Co., Ltd.]
MDI: 4,4'-diphenylmethane diisocyanate [manufactured by Tosoh Corporation]
TDI: toluene diisocyanate (2,4-, 2,6-mixture) [manufactured by Tokyo Chemical Industry Co., Ltd.]
EG: ethylene glycol [manufactured by Tokyo Chemical Industry Co., Ltd.]
IPDA: isophoronediamine [manufactured by Tokyo Chemical Industry Co., Ltd.]
MO: morpholine [manufactured by Tokyo Chemical Industry Co., Ltd.]
TBT: tetrabutoxytitanate [manufactured by Tokyo Chemical Industry Co., Ltd.]
MEK: methyl ethyl ketone [manufactured by FUJIFILM Wako Pure Chemical Corporation]
IPA: isopropyl alcohol [manufactured by FUJIFILM Wako Pure Chemical Corporation]

### [Example 1] Production of Polyurethane Resin (U-1)

A separable flask equipped with a thermocouple, a condenser, and a stirrer was set over a 60°C oil bath. In this separable flask, 60.0 g of PEG2000 preheated to 80°C, 40.0 g of NL2030DB, 3.1 g of EG, and 44.5 g of IPDI were placed. The separable flask was purged with nitrogen atmosphere, 0.01 g of TBT was added while stirring the raw material mixture at 60 rpm, and the temperature was raised to 110°C in about 1 hour.

After the temperature reached 110°C, the resulting reaction solution was stirred for about 5 hours, and it was confirmed that the NCO% of the reaction solution had reached a theoretical value, after which the reaction solution was cooled. After the reaction solution was cooled to 80°C, 304 g of MEK was added to the reaction solution, and the reaction solution was stirred to homogeneity. Once the reaction solution became homogeneous, 0.044 g of malic acid (manufactured by FUJIFILM Wako Pure Chemical Corporation) was added to the reaction solution, and 76.0 g of IPA was subsequently added, after which the reaction solution was stirred to homogeneity.

Thereafter, 15.33 g of IPDA was added to the reaction solution, and the temperature was raised to 50°C in about 1 hour. One hour after this temperature rise, IPDA was added to the reaction solution in small amounts to adjust the viscosity of the reaction solution. The total amount of the additionally added IPDA was 0.20 g. Then, 0.78 g of MO was added to the reaction solution.

After the addition of MO, the reaction solution was further stirred at 50°C for 1 hour to obtain a polyurethane resin composition having a viscosity of 67,700 mPa·s and a solid concentration of 30.7% by weight.

The results of evaluating the properties of this polyurethane resin composition and the physical properties of the polyurethane resin are shown in Table 4.

### [Examples 2 to 5 and Examples 7 to 10] Production of Polyurethane Resins (U-2, U-3, U-4, U-5, U-7, U-8, U-9, and U-10)

Polyurethane resin compositions were obtained in the same manner as in Example 1, except that the amounts of the raw materials were changed as shown in Table 1 or 2. The results of evaluating the properties of the thus-obtained polyurethane resin compositions and the physical properties of the polyurethane resins are shown in Tables 4 and 5.

### [Example 6] Production of Polyurethane Resin (U-6)

A separable flask equipped with a thermocouple, a condenser, and a stirrer was set over a 60°C oil bath. In this separable flask, 100.0 g of PEG1000 preheated to 60°C and 8.6 g of TDI were placed. The separable flask was purged with nitrogen atmosphere, 0.01 g of TBT was added with stirring at 60 rpm, and the temperature was raised to 80°C in about 1 hour.

About 2 hours after the temperature reached 80°C, infrared absorption spectrometry was performed, and the disappearance of terminal NCO groups was confirmed from the peak at 2,270 cm⁻¹.

The thus-obtained polyoxyethylene diol (Ac) had a hydroxyl value of 52.2.

After the disappearance of terminal NCO groups was confirmed, the reaction solution was cooled, and 27.2 g of NL2030DB, 4.0 g of EG, and 57.2 g of IPDI were added to the reaction solution. The separable flask was purged with nitrogen atmosphere, and the reaction solution was heated to 110°C in about 1 hour with stirring at 60 rpm.

After the temperature reached 110°C, the reaction solution was stirred for about 2 hours, and it was confirmed that the NCO% of the reaction solution had reached a theoretical value, after which the reaction solution was cooled. After the reaction solution was cooled to 80°C, 408.7 g of MEK was added to the reaction solution, and the reaction solution was stirred to homogeneity. Once the reaction solution became homogeneous, 0.059 g of malic acid (manufactured by FUJIFILM Wako Pure Chemical Corporation) was added to the reaction solution, and 102.2 g of IPA was subsequently added, after which the reaction solution was stirred to homogeneity.

Thereafter, 19.73 g of IPDA was added to the reaction solution, and the temperature was raised to 50°C in about 1 hour. One hour after this temperature raise, IPDA was added to the reaction solution in small amounts to adjust the viscosity of the reaction solution. The total amount of the additionally added IPDA was 1.03 g. Then, 1.19 g of MO was added to the reaction solution.

After the addition of MO, the reaction solution was further stirred at 50°C for 1 hour to obtain a polyurethane resin composition having a viscosity of 98,500 mPa·s and a solid concentration of 30.1% by weight.

The results of evaluating the properties of this polyurethane resin composition and the physical properties of the polyurethane resin are shown in Table 4.

### [Comparative Examples 1 to 7] Production of Polyurethane Resins (U-11 to U-17)

Polyurethane resin compositions were obtained in the same manner as in Example 1, except that the amounts of the raw materials were changed as shown in Table 3. The results of evaluating the properties of the thus-obtained polyurethane resin compositions and the physical properties of the polyurethane resins are shown in Table 6.

### [Table 1]

**Table 1**

| | | | Ex. 1 | Ex. 2 | Ex. 3 | Ex. 4 | Ex. 5 | Ex. 6 |
|---|---|---|---|---|---|---|---|---|
| Polyurethane resin (U) | | | U-1 | U-2 | U-3 | U-4 | U-5 | U-6 |
| | Polyoxyethylene diol (A) | PEG2000 | 60.0 | 70.0 | 80.0 | 80.0 | 80.0 | - |
| | Low-molecular weight Polyoxyethylene diol (a) | PEG1000 | - | - | - | - | - | 100.0 |
| | Isocyanate compound (c) | TDI | - | - | - | - | - | 8.6 |
| | Polycarbonate diol (B) | NL2030DB | 40.0 | 30.0 | 20.0 | 20.0 | 20.0 | 27.2 |
| | | NL2000B | - | - | - | - | - | - |
| | | UH-200 | - | - | - | - | - | - |
| | | NL2010DB | - | - | - | - | - | - |
| | | NL2070DB | - | - | - | - | - | - |
| Raw materials [g] | Aliphatic isocyanate compound(C) | IPDI | 44.5 | 44.5 | 44.5 | 48.9 | 55.6 | 57.2 |
| | | HDI | - | - | - | - | - | - |
| | | H12MDI | - | - | - | - | - | - |
| | Aromatic isocyanate compound | MDI | - | - | - | - | - | - |
| | C₂₋₆ Diol (D) | EG | 3.1 | 3.1 | 3.1 | 3.7 | 4.6 | 4.0 |
| | C₂₋₂₀ Diamine (E) | IPDA | 15.33/0.20 | 15.33/0.68 | 15.33/0.68 | 18.73/0.94 | 19.16/0.64 | 19.73/1.03 |
| | Catalyst | TBT | 0.01 | 0.01 | 0.01 | 0.01 | 0.01 | 0.01 |
| | Chain terminator | MO | 0.78 | 0.78 | 0.52 | 0.95 | 0.76 | 1.19 |
| | Solvent | MEK | 304 | 304 | 304 | 323 | 336 | 409 |
| | | IPA | 76.0 | 76.0 | 76.0 | 81.0 | 84.0 | 102.2 |

### [Table 2]

**Table 2**

| | | | Ex. 7 | Ex. 8 | Ex. 9 | Ex. 10 |
|---|---|---|---|---|---|---|
| Polyurethane resin (U) | | | U-7 | U-8 | U-9 | U-10 |
| | Polyoxyethylene diol (A) | PEG2000 | 85.6 | 85.6 | 85.7 | 85.7 |
| | Low-molecular weight Polyoxyethylene diol (a) | PEG1000 | - | - | - | - |
| | Isocyanate compound (c) | TDI | - | - | - | - |
| | Polycarbonate diol (B) | NL2030DB | - | - | - | - |
| | | NL2000B | 36.7 | - | - | - |
| | | UH-200 | - | 36.7 | - | - |
| | | NL2010DB | - | - | 36.7 | - |
| | | NL2070DB | - | - | - | 36.7 |
| Raw Materials [g] | Aliphatic isocyanate compound(C) | IPDI | 54.6 | 54.6 | 54.4 | 54.5 |
| | | HDI | - | - | - | - |
| | | H12MDI | - | - | - | - |
| | Aromatic isocyanate compound | MDI | - | - | - | - |
| | C₂₋₆ Diol (D) | EG | 3.8 | 3.8 | 3.8 | 3.8 |
| | C₂₋₂₀ Diamine (E) | IPDA | 18.55/0.37 | 18.77/0.98 | 18.42/1.00 | 17.73/1.06 |
| | Catalyst | TBT | 0.01 | 0.01 | 0.01 | 0.01 |
| | Chain terminator | MO | 0.32 | 0.12 | 0.49 | 0.62 |
| | Solvent | MEK | 374 | 374 | 374 | 374 |
| | | IPA | 93.5 | 93.5 | 93.5 | 93.5 |

### [Table 3]

**Table 3**

| | | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| Polyurethane resin (U) | | | U-11 | U-12 | U-13 | U-14 | U-15 | U-16 | U-17 |
| | Polyoxyethylene diol (A) | PEG2000 | 50.0 | 100.0 | 80.0 | 80.0 | 80.0 | 80.0 | 80.0 |
| | Low-molecular weight Polyoxyethylene diol (a) | PEG1000 | - | - | - | - | - | - | - |
| | Isocyanate compound (c) | TDI | - | - | - | - | - | - | - |
| | Polycarbonate diol (B) | NL2030DB | 50.0 | - | 20.0 | 20.0 | 20.0 | 20.0 | 20.0 |
| | Aliphatic isocyanate compound(C) | IPDI | 44.5 | 44.5 | 66.7 | - | 22.2 | 22.2 | - |
| | | HDI | - | - | - | - | - | 16.8 | - |
| Raw materials [g] | | H12MDI | - | - | - | 52.4 | 26.2 | - | - |
| | Aromatic isocyanate compound | MDI | - | - | - | - | - | - | 50.05 |
| | C₂₋₆ Diol (D) | EG | 3.1 | 3.1 | 6.2 | 3.1 | 3.1 | 3.1 | 3.1 |
| | C₂₋₂₀ Diamine (E) | IPDA | 15.33/0.20 | 15.33/1.19 | 22.99/1.02 | 15.33/- | 15.33/0.68 | 15.33/0.68 | - |
| | Catalyst | TBT | 0.01 | 0.01 | 0.01 | 0.01 | 0.01 | 0.01 | 0.01 |
| | Chain terminator | MO | 0.78 | 0.26 | 1.57 | - | 1.04 | 1.04 | - |
| | Solvent | MEK | 304 | 304 | 370 | 319 | 315 | 297 | 316 |
| | | IPA | 76.0 | 76.0 | 93.0 | 80.0 | 79.0 | 74.2 | 79.0 |

### [Table 4]

**Table 4**

| | | | Ex. 1 | Ex. 2 | Ex. 3 | Ex. 4 | Ex. 5 | Ex. 6 |
|---|---|---|---|---|---|---|---|---|
| Polyurethane resin (U) | | | U-1 | U-2 | U-3 | U-4 | U-5 | U-6 |
| Composition | (A)/((A)+(B)) | | 0.60 | 0.70 | 0.80 | 0.80 | 0.80 | 0.80 |
| | ([OH](A)+[OH](B))/[OH](D) | | 1.0 | 1.0 | 1.0 | 1.2 | 1.5 | 1.0 |
| | [NCO]/[OH] | | 2.0 | 2.0 | 2.0 | 2.0 | 2.0 | 2.0 |
| | [NCO]/[NH₂] | | 2.0 | 2.0 | 2.0 | 2.0 | 2.0 | 2.0 |
| | Viscosity of Polyurethane resin composition | [mPa·s] | 111,500 | 78,000 | 68,100 | 112,300 | 70,200 | 98,500 |
| | Solid concentration of Polyurethane resin composition | [wt%] | 30.2 | 30.3 | 30.8 | 30.1 | 30.0 | 30.1 |
| | Molecular weight of Polyurethane resin (U) | Mw | 145,300 | 93,700 | 114,000 | 148,000 | 115,000 | 142,100 |
| | | Mw/Mn | 2.3 | 2.4 | 2.4 | 2.3 | 2.4 | 2.3 |
| | Strength (100%M) | [MPa] | 5.2 | 4.6 | 3.1 | 4.7 | 5.3 | 4.0 |
| Evaluation | | Result | S | A | A | A | S | A |
| | Tensile strength (breaking strength) | [MPa] | 43.5 | 45.8 | 38.5 | 28.4 | 27.6 | 37.2 |
| | | Result | S | S | S | S | S | S |
| | Moisture permeability | [g/m²/24h] | 179,200 | 166,200 | 183,100 | not measured | 177,400 | 148,200 |
| | | Result | S | S | S | - | S | S |
| | Storage stability | Result | S | S | S | S | S | S |
| | Coatability | Result | S | S | S | S | S | S |

### [Table 5]

**Table 5**

| | | | Ex. 7 | Ex. 8 | Ex. 9 | Ex. 10 |
|---|---|---|---|---|---|---|
| Polyurethane resin (U) | | | U-7 | U-8 | U-9 | U-10 |
| Composition | (A)/((A)+(B)) | | 0.70 | 0.70 | 0.70 | 0.70 |
| | ([OH](A)+[OH](B))/[OH](D) | | 1.0 | 1.0 | 1.0 | 1.0 |
| | [NCO]/[OH] | | 2.0 | 2.0 | 2.0 | 2.0 |
| | [NCO]/[NH₂] | | 2.0 | 2.0 | 2.0 | 2.0 |
| | | | | | | |
| | Viscosity of Polyurethane resin composition | [mPa·s] | 69,300 | 24,260 | 120,600 | 180,200 |
| | Solid concentration of Polyurethane resin composition | [wt%] | 29.8 | 30.6 | 30.0 | 30.2 |
| | Molecular weight of Polyurethane resin (U) | Mw | 144,200 | 77,800 | 125,800 | 170,200 |
| | | Mw/Mn | 2.60 | 2.32 | 2.47 | 2.62 |
| | Strength (100%M) | [MPa] | 8.4 | 8.3 | 6.5 | 6.7 |
| Evaluation | | Result | S | S | S | S |
| | Tensile strength (breaking strength) | [MPa] | 22.1 | 25.3 | 24.8 | 24.6 |
| | | Result | A | S | A | A |
| | Moisture permeability | [g/m²/24h] | 212,000 | 152,000 | 223,000 | 174,000 |
| | | Result | S | S | S | S |
| | Storage stability | Result | B | A | S | S |
| | Coatability | Result | A | B | S | S |

### [Table 6]

**Table 6**

| | | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| Polyurethane resin (U) | | | U-11 | U-12 | U-13 | U-14 | U-15 | U-16 | U-17 |
| Composition | (A)/((A)+(B)) | | 0.50 | 1.00 | 0.80 | 0.80 | 0.80 | 0.80 | 0.80 |
| | ([OH](A)+[OH](B))/[OH](D) | | 1.0 | 1.0 | 2.0 | 1.0 | 1.0 | 1.0 | 1.0 |
| | [NCO]/[OH] | | 2.0 | 2.0 | 2.0 | 2.0 | 2.0 | 2.0 | 2.0 |
| | [NCO]/[NH₂] | | 2.0 | 2.0 | 2.0 | 2.0 | 2.0 | 2.0 | 2.0 |
| | Viscosity of Polyurethane resin composition | [mPa·s] | 67,700 | 68,000 | 77,100 | | 97,000 | 97,400 | |
| | Solid concentration of Polyurethane resin composition | [wt%] | 30.7 | 29.7 | 29.8 | | 30.1 | 30.8 | |
| | Molecular weight of Polyurethane resin (U) | Mw | 109,000 | 123,000 | 118,000 | | 78,600 | 147,800 | |
| | | Mw/Mn | 2.3 | 2.2 | 2.3 | | 1.5 | 4.0 | |
| | Strength (100%M) | [MPa] | 5.2 | 2.7 | 7.4 | | 4.3 | 1.4 | |
| Evaluation | | Result | S | B | S | gelled by addition of IPDA | A | B | gelled by addition of IPDA |
| | Tensile strength (breaking strength) | [MPa] | 41.4 | 31.6 | 27.7 | | 16.0 | 8.0 | |
| | | Result | S | S | S | | B | B | |
| | Moisture permeability | [g/m²/24h] | 61,700 | 186,900 | 65,400 | | 176,000 | not measured | |
| | | Result | B | S | B | | S | - | |
| | Storage stability | Result | S | S | S | | S | S | |
| | Coatability | Result | S | S | S | | S | S | |

As shown in Tables 4 and 5, it was found that the polyurethane resins contained in the polyurethane resin compositions obtained in Examples 1 to 10 had an excellent balance between the moisture permeability and the strength, and were soluble in a solvent having MEK as a main component.

Further, it was confirmed that these polyurethane resins are soluble in a solvent typified by MEK that has a low burden on the human body and the environment, and therefore, the use of a solvent typified by DMF that has a high burden on the human body and the environment is not necessary in the production process of these polyurethane resins and the production process of a moisture-permeable waterproof fabric.

On the other hand, as shown in Table 6, it was found that, although the polyurethane resins contained in the polyurethane resin compositions obtained in Comparative Examples 1 and 3 exhibited excellent strength and tensile strength and were soluble in a solvent having MEK as a main component, these polyurethane resins were insufficient in terms of moisture permeability.

Further, it was found that, although the polyurethane resin contained in the polyurethane resin composition obtained in Comparative Example 2 exhibited excellent moisture permeability and was soluble in a solvent having MEK as a main component, this polyurethane resin was insufficient in terms of strength. The polyurethane resins obtained in Comparative Examples 5 to 6 exhibited excellent moisture permeability and were soluble in a solvent system having MEK as a main component; however, it was found that these polyurethanes did not have a sufficient tensile strength.

Moreover, it was found that the polyurethane resins obtained in Comparative Examples 4 and 7 were not dissolved in a solvent system having MEK as a main component and were gelled during the production process. In other words, it was found that these polyurethane resins cannot be produced using a solvent typified by MEK that has a low burden on the human body and the environment, and therefore, in the production process of these polyurethane resins and the production process of a moisture-permeable waterproof fabric, it is necessary to use a solvent typified by DMF that has a high burden on the human body and the environment.

## Claims

1. A polyurethane resin composition, comprising:
a polyurethane resin (U); and
an organic solvent (S),
wherein
the polyurethane resin (U) comprises a structural unit derived from a polyoxyethylene diol (A), a structural unit derived from a polycarbonate diol (B), a structural unit derived from an aliphatic isocyanate compound (C), a structural unit derived from a diol (D) having 2 to 6 carbon atoms, and a structural unit derived from a diamine (E) having 2 to 20 carbon atoms, and
the structural unit derived from the aliphatic isocyanate compound (C) comprises a structural unit derived from an acyclic aliphatic diisocyanate (c1) in which two isocyanate groups are directly bonded to an acyclic hydrocarbon having an odd number of carbon atoms, or a structural unit derived from an alicyclic diisocyanate (c2) in which the number of isocyanate groups directly bonded to an alicyclic hydrocarbon is 0 or 1.

2. The polyurethane resin composition according to claim 1, wherein, in the polyurethane resin (U), a weight ratio (A)/((A) + (B)) of the structural unit derived from the polyoxyethylene diol (A) relative to a total weight of the structural unit derived from the polyoxyethylene diol (A) and the structural unit derived from the polycarbonate diol (B) is from 0.50 to 0.90.

3. The polyurethane resin composition according to claim 1, wherein a molar ratio of isocyanate group-derived nitrogen atoms in the structural unit derived from the aliphatic isocyanate compound (C), relative to a number of moles of amino group-derived nitrogen atoms in the diamine (E), is from 1.5 to 2.5.

4. The polyurethane resin composition according to claim 1, wherein the structural unit derived from the polyoxyethylene diol (A) comprises a structural unit derived from a polyoxyethylene diol having a hydroxyl value of from 26.0 to 140.0 mgKOH/g.

5. The polyurethane resin composition according to claim 1, wherein the structural unit derived from the polycarbonate diol (B) comprises a structural unit derived from a polycarbonate diol having a hydroxyl value of from 32.0 to 124.7 mgKOH/g.

6. The polyurethane resin composition according to claim 1, wherein
the structural unit derived from the polycarbonate diol (B) comprises a structural unit represented by the following Formula (b1) and a structural unit represented by the following Formula (b2), or the structural unit represented by the following Formula (b2), and when both the structural units of Formula (b1) and Formula (b2) are present,
a molar ratio (b1)/(b2) is from 95/5 to 5/95:
-O-R¹-O- (b1)
-O-R²-O- (b2)
in Formula (b1), R¹ represents a divalent alkylene group having 3 to 5 carbon atoms which may have a substituent; and, in Formula (b2), R² represents a divalent alkylene group having 6 to 20 carbon atoms which may have a substituent.

7. The polyurethane resin composition according to claim 1, wherein a content of the structural unit derived from the aliphatic isocyanate compound (C) in the polyurethane resin (U) is 50% by weight or less.

8. The polyurethane resin composition according to claim 1, wherein the diamine (E) comprises a diamine having 6 to 12 carbon atoms.

9. The polyurethane resin composition according to claim 1, wherein, in the polyurethane resin (U), a molar ratio of hydroxy group-derived oxygen atoms of the polyoxyethylene diol (A) and hydroxy group-derived oxygen atoms of the polycarbonate diol (B) relative to a number of moles of hydroxy group-derived oxygen atoms of the diol (D) is from 0.5 to 1.5.

10. The polyurethane resin composition according to claim 1, wherein, in the polyurethane resin (U), a molar ratio of isocyanate group-derived nitrogen atoms of the aliphatic isocyanate compound (C) relative to a total number of moles of hydroxy group-derived oxygen atoms of the polyoxyethylene diol (A), hydroxy group-derived oxygen atoms of the polycarbonate diol (B), and hydroxy group-derived oxygen atoms of the diol (D) is from 1.5 to 4.0.

11. The polyurethane resin composition according to claim 1, wherein a content of a polar solvent (Y) satisfying the following conditions (y1) and (y2) in the organic solvent (S) is 5% by weight or less:
(y1) Not classified into the carcinogenicity category according to the GHS (9th revised edition) classification; and
(y2) The acute toxicity is classified into Categories 1 to 3 according to the GHS (9th revised edition) classification, or the reproductive toxicity is classified into Category 1A or 1B according to the GHS (9th revised edition) classification.

12. The polyurethane resin composition according to claim 1, wherein the organic solvent (S) comprises a monohydric alcohol having 5 or fewer carbon atoms, and at least one selected from the group consisting of a ketone having 6 or fewer carbon atoms and an ester having 8 or fewer carbon atoms.

13. The polyurethane resin composition according to claim 1, wherein a content of the polyurethane resin (U) is from 5 to 50% by weight.

14. The polyurethane resin composition according to claim 1, further comprising a biomass-derived compound.

15. A moisture-permeable waterproof fabric comprising the polyurethane resin composition according to any one of claims 1 to 14.

16. A sportswear comprising the polyurethane resin composition according to any one of claims 1 to 14.

17. A rainwear comprising the polyurethane resin composition according to any one of claims 1 to 14.

18. A ten comprising the polyurethane resin composition according to any one of claims 1 to 14.

19. A hygiene product comprising the polyurethane resin composition according to any one of claims 1 to 14.
